## Europäisches Patentamt
## European Patent Office
## Office européen des brevets

(19)

(11) Numéro de publication: **0 098 181**
**B1**

(12) # FASCICULE DE BREVET EUROPÉEN

(45) Date de publication du fascicule du brevet:
23.07.86

(21) Numéro de dépôt: **83401093.6**

(22) Date de dépôt: **31.05.83**

(51) Int. Cl.⁴: **B 01 J 21/04,** C 10 G 35/09,
C 10 G 47/14, C 07 C 5/41,
C 07 C 5/22, C 07 C 4/18,
C 07 C 4/20, B 01 J 23/54,
B 01 J 23/89, B 01 J 37/00

(54) Procédé de fabrication d'un catalyseur de conversion d'hydrocarbures.

(30) Priorité: **25.06.82 FR 8211341**

(43) Date de publication de la demande:
**11.01.84 Bulletin 84/2**

(45) Mention de la délivrance du brevet:
**23.07.86 Bulletin 86/30**

(84) Etats contractants désignés:
**BE DE GB IT NL**

(56) Documents cité:
**DD-A-150 382**
**FR-A-2 204 676**
**GB-A-552 053**
**GB-A-1 590 436**
**US-A-2 274 634**
**US-A-2 371 237**
**US-A-2 920 053**

**CHEMICAL ABSTRACTS, vol. 69, no. 4, 22 juillet
1968, page 1268, colonne 2, no. 13306d, Columbus,
Ohio, USA**

(73) Titulaire: **SOCIETE FRANCAISE DES PRODUITS
POUR CATALYSE PRO- CATALYSE, 4, avenue de
Bois Préau, F-92502 Rueil Malmaison (FR)**

(72) Inventeur: **Blanchard, Gilbert, 5, allée des Acacias
Lagny le Sec, F-60330 Le Plessis Belleville (FR)**
Inventeur: **Dupin, Thierry, 7, rue Robert de
Luzarches, F-95140 Garges Les Gonesses (FR)**
Inventeur: **Franck, Jean- Pierre, 24, rue Ivan
Tourgueneff, F-78380 Bougival (FR)**

EP 0 098 181 B1

**0 098 181**

**Description**

La présente invention concerne des procédés de fabrication de catalyseurs pouvant être utilisés pour les réactions de conversions d'hydrocarbures et notamment d'hydroconversions d'hydrocarbures. On citera, en particulier les procédés de reformage (ou reforming) catalytique ainsi que les procédés catalytiques de fabrication d'hydrocarbures aromatiques, procédés effectués par exemple à une température comprise entre 430 et 600°C, sous une pression absolue comprise entre 0,1 et 5 MPa, (0,1 MPa $\neq$1 kg/cm²) avec une vitesse horaire comprise entre 0,1 et 10 volumes de charge liquide par volume de catalyseur, le rapport molaire hydrogène/hydrocarbures étant compris entre 1 et 20.

Les catalyseurs préparés selon l'invention conviennent aussi pour les réactions d'hydrocraquage qui sont généralement effectuées à une température comprise entre environ 260 et 530°C et sous une pression comprise entre environ 0,8 et 25 MPa. Les conditions de conversion comprennent une vitesse spatiale horaire du liquid, ou VSHL, ou volume par heure de charge liquide à 15°C par volume de catalyseur, d'environ 0,1 à 10,0, ayant de préférence une limite supérieure de 4,0 environ et un débit de circulation d'hydrogène d'environ 1 à 20 moles/mole de charge.

Les catalyseurs de l'invention conviennent également pour les réactions d'isomérisation d'hydrocarbures aromatiques (xylènes par exemple) réactions qui sont généralement effectuées à une température comprise entre environ 200 et 600°C, sous une pression comprise entre environ 0,005 et 7 MPa, le débit volumétrique horaire étant compris entre 0,1 et 10 fois le volume de catalyseur.

Les catalyseurs de l'invention conviennent encore pour les isomérisations en atmosphère d'hydrogène des hydrocarbures saturés comportant 4 à 7 atomes de carbone, à une température comprise entre 50 et 250°C, par exemple 100—200°C. On opère de préférence, sous une pression de 0,5 à 10 MPa, avec une vitesse spatiale de 0,2 à 10 litres de charge par litre de catalyseur et par heure. Le rapport molaire $H_2$/hydrocarbures est compris, par exemple, entre 0,01:1 et 10:1.

Les catalyseurs de l'invention conviennent également pour les réactions d'hydrodéalkylation d'hydrocarbures aromatiques ou de déalkylation à la vapeur d'eau d'hydrocarbures aromatiques, ces réactions étant effectuées dans les conditions opératoires connues, généralement entre 300 et 600°C, pour fabriquer par exemple du benzène à partir de toluène ou à partir d'autres alkylbenzènes.

Les catalyseurs utilisés dans les réactions énumérées ci-dessuss sont constitués d'une matrice ou d'un support à base d'alumine sur lequel a été introduite une phase dite active à base d'au moins un métal noble du groupe VIII (famille du platine), d'un métal additionnel ou promoteur appartenant à la famille du platine ou appartenant à un autre groupe de la classification périodique des éléments et un halogène.

Ainsi, les catalyseurs spécifiques appropriès au procédé selon l'invention, sont les catalyseurs renfermant un support d'alumine, des teneurs critique de divers éléments métalliques adéquats (métaux ou composés de métaux) et un halogène. Ainsi les catalyseurs spécifiques de reforming ou de production d'hydrocarbures aromatiques ou des autres réactions mentionnées plus haut, renferment généralement en poids par rapport à la matrice ou au support d'alumine: (par la suite on utilisera le terme général "support")

a) 0,02 à 1% d'au moins un premier métal choisi parmi le platine, le palladium, l'iridium, le ruthénium, le rhodium et l'osmium (et de préférence le platine, l'iridium, le ruthénium et le rhodium),

b) 0,01 à 2 % d'au moins un métal additionnel ou promoteur choisi par exemple, parmi un métal noble de la famille du platine et parmi le titane, le rhénium, l'étain, le germanium, l'indium, le thallium, le manganèse, le nickel, le fer, le cobalt, le zinc, le cuivre, l'argent, l'or, le niobium, le lanthane, le cérium, le samarium, le zirconium, le thorium, l'hafnium, le plomb, le sélénium, le gallium, le vanadium, le technétium et l'uranium, et

c) 0,1 à 10% d'au moins un halogène, par exemple le chlore ou le fluor, pour des réactions telles qui reformage, production d'hydrocarbures aromatiques, certaines isomérisations etc.

Comme catalyseurs préférés pour la présente invention, on citera plus particulièrement les catalyseurs renfermant:

— du platine et de l'iridium,
— du platine et au choix au moins l'un des métaux choisi dans le groupe constitué par le titane, le rhénium, l'étain, le germanium, l'indium, le thallium, le manganèse, le nickel, le fer, le cobalt, le zinc, le cuivre, l'or, l'argent, le niobium, le lanthane, le cérium, le samarium, le zirconium, le thorium, l'hafnium, le plomb, le gallium, le vanadium, le technetium, l'uranium et le sélénium
— du platine, de l'iridium et de l'étain,
— du platine, de l'iridium et du germanium,
— du platine, de l'iridium et du sélénium,
— du platine, de l'iridium et du thallium,
— du platine, de l'iridium et de l'indium,
— du platine, de l'iridium et du titane,
— du platine, de l'iridium et du rhénium,
— du platine, de l'iridium et du manganèse,
— du platine, de l'iridium et du cuivre ou de l'or ou de l'argent,

2

— du platine, du rhénium et au moins l'un des divers métaux énumérés ci-dessus (par exemple le sélénium ou tout autre promoteur),

— du platine, du germanium et au moins l'un des divers métaux énumérés précédemment,

— du platine, de l'étain et au moins l'un des divers métaux énumérés précédemment,

— du platine, de l'indium ou du thallium et au moins l'un des divers métaux énumérés précédemment.

Dans l'art antérieur, les méthodes de préparation consistent à introduire la phase active sur le support prêt à l'emploi, acheté dans le commerce.

Le support a été préparé par toute méthode adéquate:

Par exemple, comme supports de catalyseurs, on peut utiliser des agglomérés d'alumine spécialement résistants et poreux. On part d'une alumine telle qui l'hydroargillite et l'on procède à une activation par déshydratation rapide et partielle de cet hydrate. On confère ainsi à l'alumine obtenu la possibilité d'être agglomérée, par exemple sous forme de billes, après avoir été humectée d'eau. On procède ensuite à un mûrissement destiné à augmenter la dureté et on termine par une réactivation par chauffage, l'ensemble de ces opérations conduit selon les conditions, à des agglomérés de diverses caractéristiques.

On a également propose d'obtenir des agglomérés poreux d'alumine active à partir de gels d'alumine. Dans le brevet français No. 1 438 497, le procédé consiste à sécher très rapidement une bouillie d'alumine hydratée amorphe dans des conditions telles qui l'état amorphe de la poudre fine ainsi obtenu soit maintenu, à humecter d'eau cette alumine en poudre, ou préférentiellement d'une solution ammoniacale, à l'agglomérer, à faire mûrir les agglomérés obtenus en atmosphère confinée à température peu élevée, comprise préférentiellement entre 60 et 100°C, à sécher ces agglomérés puis à les calciner à une température en relation avec la surface active et la porosité désirées.

D'une façon générale, lorsqu'on utilise un support d'alumine, celui ci peut être préparé synthétiquement ou être une matière naturelle. Quel que soit le type d'alumine utilisé, il doit être activé avent l'utilisation par un ou plusieurs traitements, tels qui séchage, calcination, traitement à la vapeur, et il peut se présenter généralement sous la forme d'agglomérés d'alumine de transition connue sous le nom d'alumine activée, d'alumine poreuse ou de gel d'alumine activée.

Ainsi, par exemple, le support d'alumine peut être préparé en ajoutant un réactif alcalon convenable, tel que l'hydroxyde d'ammonium, à un sel d'aluminium, tel que le chlorure d'aluminium ou le nitrate d'aluminium pour former un gel d'hydroxyde d'aluminium qui, par séchage et calcination, est transformé en alumine. Le support d'alumine peut être mis sous toutes formes désirées, telles qui sphères, pilules, gâteaux, extrudats, poudres ou grains, et utilisé à toutes tailles désirées. On préfère en général des sphères d'alumine. Celles-ci peuvent être fabriquées en continu par la méthode connue de la goute dans l'huile, qui consiste à former un hydrosol d'alumine, à associer l'hydrosol avec un agent gélifiant convenable, et à faire tomber le mélange obtenu dans un bain d'huile maintenu à température élevée. Les gouttelettes restent dans le bain d'huile jusqu'à ce qu'elles forment des sphères d'hydrogel. On lave ensuite les sphères, les sèche et calcine. Les sphères finales sont de l'alumine gamma cristalline. Cette méthode est décrite dans US—A—2 620 314.

L'alumine que l'on préfère le plus souvent utiliser dans les catalyseurs de réformage est la gamma-alumine. Il existe divers procédés d'obtention de la gamma-alumine. Bien qu'il existe divers modes de préparation d'alumine hydratée à partie de laquelle la gamma-alumine peut être préparée à son tour, l'alumine hydratée est formée le plus fréquemment comme indiqué plus haut, par préparation d'un "gel" d'alumine. On rappelle que le nom de "gel" est appliqué à des préparations d'alumines qui sont des formes hydroxylées ou oxyhydroxylées ou au moins partiellement hydratées (ayant des structures amorphes ou cristallisées) obtenues à partir de solutions de sels d'aluminium ou d'aluminates alcalins. (Le terme "sol" étant habituellement le terme appliqué à des solutions liquides colloïdales qui donnent des gels solides par déshydratation partielle). Ainsi, le brevet US—A—3 280 041 mentionne deux procédés généraux de préparation d'alumine hydratée. L'un de ces procédés donne des "alumines précipitées", c'est-à-dire qui les alumines sont formées par addition d'un acide ou d'un sel d'acide, par exemple l'acide sulfurique, l'acide chlorhydrique, sur ou à un aluminate de mètal alcalin.

Un autre type d'alumine hydratée est appelé "sol d'alumine du type Heard", et consiste en sols d'alumine préparés par digestion d'aluminium métallique par l'action d'acide acétique ou d'un acide équivalent. Ces sols et leurs procédés de préparation sont décrits par exemple dans les brevets US—A—2 258 099 et No. 2 274 634.

Un autre procédé connu de préparation de l'alumine consiste à dissoudre de l'aluminium dans un alcool pour former un alcoolate ou alkoxyde d'aluminium, lequel est hydrolysé pour former un alcool et un hydroxyde ou l'oxyhydroxyde d'aluminium. On obtient alors une pâte d'alumine hydratée. L'eau est éliminée de l'hydroxyde d'aluminium pour former de l'alumine.

Le FR—A—2 204 676 concerne un procédé dans lequel l'alumine est produite par réaction d'aluminium avec un alcool pour former un alcoolate d'aluminium. L'alumine utilisée est un sous-produit des réactions de synthèse de Ziegler pour la formation d'alcools supérieurs (Les réactions de synthèse d'alcools de Ziegler sont décrites, par exemple, dans le brevet US—A—2 892 858).

Selon l'art antérieur, on peut utiliser plusieurs méthodes pour ajouter ensuite la phase active du catalyseur dans le support:

d'une façon générale on peut incorporer dans le support chaque métal individuellement ou tous les métaux ensemble, par toute méthode adéquate à savoir par coprécipitation ou cogélation avec le support poreux ou échange ionique avec lesupport gélifié ou encore imprégnation avec le support, soit avant, soit après le séchage et la calcination de ce support. Les méthodes préférées consistent à imprégner le support au moyen de solutions de composés des métaux, que l'on désire introduire. On utilise soit une solution commune de ces métaux, soit des solutions distinctes pour chaque métal ou chaque groupe de métaux. Quand on utilise plusieurs solutions, on peut procéder à des séchages et/ou calcinations intermédiaires. On termine habituellement par une calcination par exemple entre environ 500 et 1000°C, de préférence en présence d'oxygène libre, par exemple en effectuant un balayage d'air.

Comme exemples de composés de métaux autres que ceux des métaux nobles du groupe VIII (famille du platine), on peut mentionner, par exemple, les nitrates, les chlorures, les bromures, les fluorures, les acétates de ces métaux ou encore tout autre sel ou oxyde de ces métaux soluble dans l'eau ou l'acide chlorydrique (chloroplatinate par exemple). On peut encore mentionner des complexes organiques contenant ces métaux.

Lex métaux nobles du groupe VIII et en particulier le platine peuvent être utilisés sous l'une quelconque de leurs formes connues, par exemple pour le platine, l'acide hexachloroplatinique, le chloroplatinate d'ammonium, le sulfure, le sulfate ou le chlorure de platine. Le ruthénium, à titre d'exemple, peut être utilisé sous une forme connue, quelconque, par exemple sous forme de chlorure, bromure, sulfate, ou sulfure ou encore sous forme, par exemple, d'acétylacétonate, etc.

L'halogène peut provenir de l'un des halogénures ci-dessus ou être introduit sous forme d'acide chlorhydrique ou d'acide fluorhydrique, de chlorure d'ammonium, de fluorure d'ammonium, de chlore gazeux, ou d'halogénure d'hydrocarbure, par exemple $CCl_4$, $CH\,Cl_3$, ou $CH_3\,Cl$.

Une première méthode de préparation consiste par exemple à imprégner le support au moyen d'une solution aqueuse de nitrate ou autre composé d'un métal autre qu'un métal de la famille du platine, sécher vers 90 à 150°C et calciner sous air quelques heures à une température comprise entre 400 et 1200°C; ensuite suivra une deuxième imprégnation au moyen d'une solution renfermant au moins un métal de la famille du platine (par exemple au moyen d'une solution d'acide hexachloroplatinique).

Une autre méthode consiste par exemple à imprégner le support au moyen d'une solution renfermant à la fois

1) le ou les métaux de la famille du platine (acide hexachloroplatinique par exemple),

2) Le ou les métaux autres que ceux de la famille du platine (par exemple un chlorure, un bromure, un fluorure, un sulfate ou un acétate du métal choisi ou encore tout autre sel du métal choisi soluble dans l'eau ou l'acide chlorhydrique ou autre solvant approprié (chloroplatinate, acétylacétonate par exemple) et

3) éventuellement, du chlore ou du fluor.

Une autre méthode encore, consiste à introduire les éléments métalliques en effectuant autant d'imprégnations successives qu'il y a d'éléments métalliques dans le catalyseur; par exemple, on introduit:

— d'abord un métal de la famille du platine au moyen d'une solution le contenant, cette étape est suivie ou non d'un séchage et d'une calcination,

— puis le ou les autres métaux de la famille du platine (si le catalyseur en comporte plusieurs) au moyen d'une solution le ou les contenant;

— et enfin le ou les autres métaux non nobles, cette dernière imprégnation étant suivie de séchage et calcination à une température comprise par exemple entre environ 400 et 1200°C.

Il est bien entendu que l'ordre des imprégnations donné ci-dessus n'est pas obligatoire et peut être différent.

Les catalyseurs ainsi obtenus dans l'art antérieur, qui sont peu à peu perfectionnés les uns par rapport aux autres par l'utilisation de tel ou tel métal promoteur utilisé avec telle ou telle concentration critique, ont permis d'obtenir des résultats souvent remarquables dans les réactions de conversion d'hydrocarbures pour ce qui concerne en particulier les rendements et la sélectivité de la réaction ainsi que la stabilité, donc la durée de vie, du catalyseur.

Mais on a maintenant constaté qui l'on peut encore augmenter d'une part le rendement et la sélectivité de la réaction pour laquelle il est conçu et d'autre part la stabilité et durée de vie de ce catalyseur. Ces améliorations sont dues à une méthode particulière de préparation des catalyseurs. Cette méthode permet en particulier d'utiliser les catalyseurs dans des conditions opératoires sévères. Ainsi, plus particulièrement, l'utilisation des catalyseurs préparés conformément à la présente invention s'applique, à titre d'exemples aux réactions de reformage en vue d'obtenir une essence d'indice d'octane clair élevé supérieur ou égal à 100, par exemple, ou d'obtenir des hydrocarbures aromatiques. Les conditions sévères des réactions d'hydroreforming ou d'hydroreformage catalytique sont plus particulièrement les suivantes: la température moyenne est comprise entre, environ 480 et 580°C, la pression est comprise entre, environ 0,5 et 1,8 MPa, (5 et 18 kg/cm²) de préférence 0,6 et 1,3 MPa, la vitesse horaire est comprise entre 1 et 10 volumes de charge liquide par volume de catalyseur et le taux de recyclage est compris entre 4 et 10 moles d'hydrogène par mole de charge. La charge est généralement un naphta distillant entre environ 60°C et environ 200°C, en particulier un naphta de distillation directe.

La présente invention concerne un procédé de fabrication de catalyseur. Le procédé consiste à préparer le support du catalyseur soit par une méthode d'extrusion, soit par une méthode incluant

4

# 0 098 181

l'utilisation d'un drageoir tournant ou équipement équivalent, et est caractérisé en ce que l'on confère à la poudre d'alumine utilisée un certain taux de peptisation et en ce que simultanément une partie au moins de la phase active du catalyseur est introduite au cours de la peptisation d'au moins une partie de la poudre d'alumine.

L'invention est limitée à des catalyseurs à support d'alumine. Toutes les alumines ne conviennent pas. Celles qui conviennent doivent posséder un taux de peptisation (dispersion) qui doit être de l'ordre d'au moins 10%. Conformément à l'invention, on peptise cette alumine jusqu'à un taux de peptisation qui sera compris de préférence entre 10 et 30%. Cela signifie qui sur 100 grammes de la poudre utilisée, 10 grammes au moins de ce support sont dispersables dans une solution aqueuse ou acide dont le pH est inférieur ou égal à 4 environ (On entend par alumine dispersable une alumine qui peut former une solution colloïdale stable).

Dans une mise en oeuvre préféré de la présente invention, l'alumine utilisée est produite par réaction entre de l'aluminium et un alcool en vue de former un alcoolate d'aluminium. L'alumine ainsi utilisée est un sous-produit des réactions de synthèse de Ziegler pour la formation d'alcools supérieurs. Les réactions de synthèse d'alcools de Ziegler sont connues et décrites notamment dans US—A2 892 858 et No. 2 276 278; la dernière étape du procédé est une étape d'hydrolyse des alcoolates d'aluminium au cours de laquelle on obtient des alcools. Par élimination de l'eau de la pâte d'hydroxyde d'aluminium, on récupère une alumine de grande pureté. Le brevet US—A—3 852 190 enseigne de calciner cette alumine et d'y introduire, soit après, soit avant la calcination, un métal noble de la famille du platine et éventuellement un promoteur tel que le rhénium et d'utiliser le catalyseur ainsi obtenu dans des réactions d'hydroconversion d'hydrocarbures, par exemple le reformage.

Conformément à la présente invention, on a découvert qu'on obtenait des résultats améliorés dans les réactions d'hydroconversion d'hydrocarbures en procédant à une peptisation de l'alumine au moyen d'une solution aqueuse ou d'une solution aqueuse acide. Une partie de la phase active c'est-à-dire le ou les métaux ou composés de métaux que l'on se propose d'introduire sur le support d'alumine) est ajoutée pendant la peptisation de l'alumine, cette partie de phase active étant ajoutée sous forme soluble soit dans l'alumine à traiter par la solution aqueuse, soit dans la solution aqueuse, soit à la fois dans l'alumine et dans la solution aqueuse.

Lorsque la phase active renferme à la fois au moins un métal précieux tel qu'un métal noble de la famille du platine (présent dans le catalyseur final prêt à l'emploi sous forme métallique) et au moins un métal promoteur (tel que le rhénium, l'indium, l'étain, le germanium, le zinc, le cuivre, l'or, l'argent, le manganèse, le thallium, le titane, le niobium, etc, présent généralement sous forme d'oxyde, il peut être avantageux d'introduire uniquement le ou les métaux promoteurs dans la solution aqueuse de peptisation ou dans l'alumine ou à la fois dans la solution aqueuse et dans l'alumine, tandis que le ou les métaux nobles seront introduits ultérieurement avant ou après la calcination de l'alumine, par toute méthode conventionelle adéquate, telle qu'une imprégnation, par exemple. Pour certains promoteurs (pour l'étain par exemple), il est avantageux d'introduire ensemble le métal précieux et le promoteur).

Lorsque la solution aqueuse de peptisation est acide, on utilise tout acide convenable, par exemple l'acide nitrique, l'acide chlorhydrique et l'acide acétique ou tout autre acide adéquat.

De préférence, conformément à la présente invention, la préparation du catalyseur est effectuée comme suit:

— Eventuellement, la poudre d'alumine utilisée comme support est lavée puis séchée par toute méthode adéquate comme, par exemple à l'étuve, de façon à obtenir une poudre caractérisée par une perte au feu de l'ordre de 2 à 40%.

— Eventuellement encore, il est, à ce stade, possible de calciner au moins partiellement la poudre séchée. Ainsi on peut soumettre, à une température comprise entre environ 300 et 800°C, 20 à 80% (en poids) de la poudre d'alumine et mélanger la poudre calcinée à la poudre non calcinée.

Ensuite on pratique la mise en forme de la poudre conformément à l'invention, soit par extrusion, soit au moyen d'un drageoir tournant ou d'un moyen équivalent, cette mise en forme étant caractérisée par l'ajoit simultané d'une partie au moins de la phase active.

Si l'on opère par extrusion, on procède de la façon suivante:

· pendant une durée comprise entre environ cinq minutes et cinq heures, on malaxe la poudre en présence d'eau ou d'eau acidulée, l'eau ou l'eau acidulée renfermant au moins en partie la phase active du catalyseur (c'est-à-dire les oxydes de métaux et/ou les métaux précieux).

On utilise une quantité d'eau, ou une quantité d'eau acidulée qui peut représenter en poids par exemple 3 % à 60 % per rapport à la masse de la poudre.

· La pâte obtenue est extrudée par toute méthode adéquate, par exemple sur extrudeuse mono vis ou double vis ou de toute autre conception, au travers d'une filière.

· Les extrudés obtenus sont séchés et éventuellement calcinés à une température généralement inférieure à 550°C, de façon à ce que la perte au feu des solides obtenus soit de l'ordre de 2 à 40%.

5

C'est à ce stade qu'on introduit éventuellement et conventionnellement la partie de la phase active qui n'a pas encore été mélangée au support et l'on peut terminer par un séchage ou calcination par exemple à une température inférieure à 550°C, voire 350°C.

- Eventuellement ensuite, les extrudés sont soumis à un traitement hydrothermal (vapeur d'eau) en milieu neutre ou acide ou basique à une température comprise entre 80 et 500°C, en vue de faire évoluer les structures amorphes en structure boehmite ou pseudoboehmite et d'améliorer les propriétés mécaniques. (Ce traitement peut d'ailleurs être effectué à la fin de la calcination décrite ci-après).
- On procède à la calcination des extrudès obtenus, à une température généralement comprise entre 350 à 1000°C.
- Un traitement final conventionnel à l'hydrogène est possible.

Si l'on opère par la méthode du granulateur tournant ou similaire (drageoir ou autre: assiette tournante, bol tournant, cylindre tournant, etc...) on procède comme suit: d'une part on fait couler la poudre sur le granulateur et d'autre part, on introduit simultanément sur le granulateur, soit en la versant soit en la vaporisant, une solution aqueuse ou une solution aqueuse acidulée qui renferme au moins en partie la phase active du catalyseur (c'est-à-dire les oxydes de métaux et/ou les métaux précieux). Généralement, on mouille le drageoir ou le granulateur, pendant que la poudre tourne. La poudre grossit par collage des particules de poudre qui sont présentes. On enlève les particules selon les méthodes habituelles, par exemple en les ejectant d'un drageoir par centrifugation puis on les sèche et on les calcine comme expliqué plus haut pour la méthode dite d'extrusion (avec introduction éventuelle, comme expliqué ci-dessus, de la partie de la phase active qui n'a pas encore été introduite sur le support).

Un perfectionnement de la méthode consisterait à envoyer sur le granulateur, une partie de la poudre (environ 0 à 40% de cette poudre) déjà diluée dans la solution aqueuse ou mieux dans la solution aqueuse acidulée. (On peut ainsi diluer une partie de la poudre dans l'acide à l'aide duquel on propose ladite solution aqueuse acidulée).

D'une façon générale, que la méthode de fabrication du catalyseur soit celle de l'extrusion ou du granulateur, leu métaux de la phase active sont introduits dans l'eau ou l'eau acidulée sous une forme soluble telle que décrite précédemment dans cette demande de brevet.

Exemple 1 (comparatif)

On prépare plusieurs catalyseurs non conformes à l'invention.

On a utilisé un support d'alumine préparé par réaction de synthése d'alcools de Ziegler conformément aux techniques connues décrites dans US—A—2 892 858 et dans un article de R. J. Butler et al. ("Production of high purity Alumina by the Hydrolysis of Aluminum Alkoxides", extrait 8—D, 16—20 mars, 1969).

On utilise ainsi une alumine du même type qui l'alumine commerciale "Catapel SB" dont la surface spéficique, après calcination est de l'ordre de 250 m²/g.

Cette alumine est extrudée après sa mise en pâte avec de l'eau et de l'acide nitrique: on prépare ainsi une pâte homogène avec 500 g d'alumine, 480 cm³ d'eau distillée et 20 cm³ d'acide nitrique 0,001 M. La pâte est alors extrudée à travers une filière de 1,5 mm, sur une extrudeuse type à piston. Les extrudès sont séchés vers 300°C jusqu'à poids constant, puis calcinés 3 heures dans le l'air sec à 650°C.

A l'aide du support d'alumine ainsi obtenu, on prépare 6 catalyseurs $A_1$ à $F_1$ renfermant, en poids, chacun 0,2% de platine et 1,18% de chlore et renfermant en outre:

|                            |                      |
| -------------------------- | -------------------- |
| pour le catalyseur $A_1$   | 0,5% de rhénium      |
| pour le catalyseur $B_1$   | 0,5% d'étain         |
| pour le catalyseur $C_1$   | 0,5% de thallium     |
| pour le catalyseur $D_1$   | 0,5% d'indium        |
| pour le catalyseur $E_1$   | 0,5% de titane       |
| pour le catalyseur $F_1$   | 0,04% d'iridium      |

Le catalyseur $A_1$ est préparé en ajoutant à 100 grammes d'alumine

— d'une part, 65 cm³ d'une solution aqueuse renfermant:
1,90 g d'acide chlorhydrique concentré (densité 1,19)
10 g de solution aqueuse d'acide chloroplatinique à 2% en poids de platine

— d'autre part,
51 cm³ d'une solution d'acide perrhénique à 0,98% en poids de rhénium.

6

On laisse en contact 10 heures puis on sèche à l'étuve à 100°C pendant 6 heures, puis on calcine sous courant d'air sec pendant 2 heures à 380°C puis pendant 2 heures à 530°C. Puis on réduit sous courant d'hydrogène sec (alumine activée) pendant 2 heures à 450°C.

Les catalyseurs $B_1$, $C_1$, $D_1$, $E_1$ et $F_1$ ont été préparés en ajoutant à 100 g d'alumine d'une part, 65 cm³ d'une solution aqueuse renfermant:

1,90 g d'acide chlorhydrique concentré (densité 1,19)
10 g de solution aqueuse d'acide chloroplatinique à 2% en poids de platine

— d'autre part, 100 cm³ d'une solution aqueuse renfermant:

2,5 g de solution d'acétate d'étain à 20% d'étain, pour le catalyseur $B_1$,
2,5 g de solution d'acétate de thallium contenant 20% en poids de thallium pour le catalyseur $C_1$,
10,75 g de solution de trichlorure de titane à 15% en poids de trichlorure de titane pour le catalyseur $E_1$ et,
1,87 g de nitrate d'indium pour le catalyseur $D_1$.
1,74 cm³ d'une solution aqueuse d'acide chloroiridique à 2,3% en poids pour le catalyseur $F_1$.

On séche, calcine et réduit les catalyseurs obtenus comme indiqué pour le catalyseur $A_1$
Ces catalyseur ont une surface spécifique de 230 m²/g

Exemple 2 (comparatif)

On prépare encore plusieurs catalyseurs $A_2$ à $F_2$ non conformes à l'invention.

On utilise, à titre de support, une alumine préparée, comme indiqué dans l'exemple 1, par réaction de synthése d'alcools de Ziegler.

Cette alumine ensuite n'est pas extrudée comme dans l'exemple 1 mais elle est agglomérée à l'aide d'un granulateur tournant en utilisant comme liquide une solution d'acide nitrique à pH2,2. Les billes obtenues sont muries en atmosphére confinée pendant 24 heures à 80°C. Elles sont ensuite séchées vers 300°C et calcinées à l'air sec à 570°C.

A l'aide de ce support d'alumine ainsi obtenu, on procéde à la préparation des catalyseurs $A_2$ à $F_2$ exactement comme il a été expliqué pour l'exemple 1.

Les catalyseurs $A_2$ à $F_2$ renfermant donc respectivement les mêmes teneurs en platine, en chlore et en métal additionnel que les catalyseurs $A_1$ à $F_1$.

Ces catalyseurs ont une surface spécifique de 235 m²/g.

Exemple 3

On prépare des catalyseurs $A_3$ à $F_3$ ayant respectivement les mêmes teneurs en platine, en chlore et en métal additionnel que les catalyseurs $A_1$ à $F_1$.

On utilise l'alumine de type Catapal SB préparée à l'exemple 1. Cette alumine est séchée à l'étuve de façon à posséder à l'issue du séchage, une perte au feu de 25%. Le matériel poudreux est lavé puis séché. Puis on calcine, à 550°C, 70% de la poudre et on mélange ensuite la poudre calcinée et la poudre non calcinée.

La poudre obtenue est extrudée après sa mise en pâte avec de l'eau et de l'acide nitrique: on prépare ainsi une pâte homogène après mélange pendant 30 mn de 500 grammes d'alumine et d'une solution qui contient:

— d'une part 250 cm³ d'eau distillée et 20 cm³ d'acide nitrique 0,001 M et
— d'autre part
· soit 260 cm³ d'une solution d'acide perrhénique à 0,98% en poids de rhénium pour le catalyseur $A_3$,
· soit 500 cm³ d'une solution aqueuse renfermant
12,5 g de solution d'acétate d'étain à 20% d'étain pour le catalyseur $B_3$,
12,5 g de solution d'acétate de thallium contenant 20% en poids de thallium pour le catalyseur $C_3$,
53,75 g de solution de trichlorure de titane à 15% en poids de trichlorure de titane pour le catalyseur $E_3$ et
9,35 g de nitrate d'indium pour le catalyseur $D_3$.
8,7 cm³ d'une solution aqueuse d'acide chloroiridique à 2,3% en poids pour le catalyseur $F_3$.

(Le taux de peptisation de l'alumine étain de l'ordre de 15% avec formation d'une solution colloïdale stable).

La pâte est extrudée à travers une filière de 1,5 mm sur la même extrudeuse que dans l'exemple 1.

Les extrudés sont séchés à 300°C de façon à ce que la perte au feu des solides obtenus soit d'environ 20%.

A la masse catalytique ainsi obtenue, on ajoute alors 235 cm³ d'une solution aqueuse contenant
9,5 g d'acide chlorhydrique concentré (densité 1,19)
et 50 g de solution aqueuse d'acide chloroplatinique à 2% en poids de platine.

7

On laisse en contact 10 heures puis on sèche à l'étuve à 100°C pendant 6 heures, puis on calcine sans courant d'air sec pendant 2 heures à 380°C puis pendant 2 heures à 530°C.

Enfin on réduit sous courant d'hydrogène sec (alumine activée) pendant deux heures à 450°C.

Ces catalyseurs ont une surface spécifique de 232 m²/g.

Exemple 4

On prépare des catalyseurs $A_4$ à $F_4$ renfermant respectivement les mêmes teneurs en platine, en chlore et en métal additionnel que les catalyseurs $A_1$ à $E_1$.

On utilise toujours l'alumine de type Catapal SB préparée à l'exemple 1.

Cette alumine est séchée à l'étuve de façon à posséder à l'issue du séchage, une perte au feu de 25%. Le matériel poudreux est lavé puis séché.

La poudre obtenue est agglomérée à l'aide d'un granulateur tournant: on fait couler progressivement 5000 grammes de la poudre sur le granulateur et pendant cette opération on introduit simultanément et progressivement sur le granulateur, en la vaporisant, une solution aqueuse renfermant:

— 2500 cm³ d'eau distillée
— 200 cm³ d'acide nitrique 0,1 M et
    soit: 255 cm³ d'une solution d'acide perrhénique à 98% en poids de rhénium, pour le catalyseur $A_4$,
    soit: 225 cm³ d'une solution aqueuse renfermant:
        12,5 g de solution d'acétate d'étain à 20% d'étain pour le catalyseur $B_4$,
        ou    12,5 g de solution d'acétate de thallium contenant 20% en poids de thallium, pour le catalyseur $C_4$,
        ou    53,15 g de solution de trichlorure de titane à 15% en poids de trichlorure de titane pour le catalyseur $E_4$,
        ou    9,35 g de nitrate d'indium pour le catalyseur $D_4$,
        ou    8,7 cm³ d'une solution aqueuse d'acide chloroiridique à 2,3% en poids pour le catalyseur $F_4$.

On mouille ainsi le drageoir ou le granulateur pendant qu'une partie de la poudre tourne.

La poudre grossit par collage des particules de poudre qui sont présentes.

On enlève les particules en les éjectant du drageoir par centrifugation puis on les sèche à 300°C de façon à ce que la perte au feu des solides obtenus soit d'environ 20%.

Le taux de peptisation de l'alumine était de l'ordre de 15% avec formation d'une solution colloïdale stable.

A 500 grammes de la masse catalytique ainsi obtenue, on ajoute alors 235 cm³ d'une solution aqueuse contenant 9,5 g d'acide chlorhydrique concentré (densité 1,19) et 50 g de solution aqueuse d'acide chloroplatinique à 2% en poids de platine.

On poursuit la fabrication du catalyseur comme dans l'exemple 3.

Ces catalyseurs ont une surface spécifique de 228 m²/g.

Exemple 5

En vue d'obtenir une essence ayant un nombre d'octane clear (ou clair) égal à 103, on se propose de traiter un naphta ayant les caractéristiques suivantes:

| | |
|---|---|
| — distillation ASTM | 80—160°C |
| — composition: hydrocarbures aromatiques | 7% en poids |
|                hydrocarbures naphténiques | 27% en poids |
|                hydrocarbures paraffiniques | 66% en poids |
| — nombre d'octane "clear research" | environ 37 |
| — poids moléculaire moyen | 110 |
| — densité à 20°C | 0,782 |

Ce naphta passe avec de l'hydrogène recyclé sur les catalyseurs $A_1$ à $E_1$, $A_2$ à $E_2$, $A_3$ à $E_3$, $A_4$ à $E_4$ et $F_1$ à $F_4$.

On opère en continu dans un réacteur à lit mobile. Les conditions opératoires sont les suivantes:

— pression                                              1 M Pa

— température                                           530°C

— rapport molaire $H_2$/hydrocarbures                   8

— volume de naphta/volume de catalyseur/heure          1,65

Le tableau I suivant indique, au bout de 200 heures, le rendement obtenu en $C_5^-$ et le pourcentage d'hydrogène contenu dans le gaz de recyclage.

On note un gain significatif en rendement et en hydrogène de recyclage lorsque le catalyseur est préparé conformément à l'invention.

TABLEAU I

| Catalyseur | Metal precieux | Metal promoteur | Rendement $C_5^-$ (poids) | Gaz recyclage % $H_2$ (molaire) |
|---|---|---|---|---|
| $A_1$ | platine | rhénium | 74,9 | 75,3 |
| $A_2$ | " | rhénium | 75,0 | 75,4 |
| $A_3$ | " | rhénium | 75,7 | 76,2 |
| $A_4$ | " | rhénium | 75,8 | 76,4 |
| $B_1$ | " | étain | 76,6 | 76,2 |
| $B_2$ | " | étain | 76,5 | 76,1 |
| $B_3$ | " | étain | 77,4 | 77,2 |
| $B_4$ | " | étain | 77,4 | 77,2 |
| $C_1$ | " | thallium | 74,9 | 75,0 |
| $C_2$ | " | thallium | 74,8 | 75,0 |
| $C_3$ | " | thallium | 75,8 | 76,0 |
| $C_4$ | " | thallium | 75,8 | 76,0 |
| $D_1$ | " | indium | 75,5 | 75,2 |
| $D_2$ | " | indium | 75,4 | 75,2 |
| $D_3$ | " | indium | 76,3 | 76,0 |
| $D_4$ | " | indium | 76,4 | 76,1 |
| $E_1$ | " | titane | 76,7 | 76,2 |
| $E_2$ | " | titane | 76,7 | 76,1 |
| $E_3$ | " | titane | 77,8 | 77,1 |
| $E_4$ | " | titane | 77,9 | 77,2 |
| $F_1$ | Platine et et iridium | | 75,2 | 74,9 |
| $F_2$ | | | 75,3 | 75,0 |
| $F_3$ | | | 76,2 | 75,8 |
| $F_4$ | | | 76,4 | 75,9 |

9

Exemple 6

Pour le catalyseur au platine et à l'étain, on répète les exemples 3 et 4 en ajoutant les 325 cm³ de la solution aqueuse contenant le platine avec la solution contenant l'étain.

Dans cette méthode donc, le métal du groupe VIII et le métal additionnel sont ajoutés ensemble dans la poudre d'alumine avant l'extrusion de l'alumine (catalyseur $B_5$) ou avant le passage de l'alumine dans le granulateur (catalyseur $B_6$). On obtient (dans les conditions de l'exemple 5) avec ces catalyseurs $B_5$ et $B_6$ des résultats légèrement supérieurs à ceux obtenus respectivement avec les catalyseurs $B_3$ et $B_4$ (Tableau II).

TABLEAU II

| Catalyseur | Metal precieux | Metal promoteur | Rendement $C_5^-$ (poids) | Gas de recyclage % $H_2$ (molaire) |
|---|---|---|---|---|
| $B_5$ | Platine | Etain | 77,6 | 77,5 |
| $B_6$ | Platine | Etain | 77,8 | 77,8 |

**Revendications**

1. Procédé de fabrication d'un catalyseur renfermant (a) une matrice ou un support à base de poudre d'alumine et (b) une phase active renfermant au moins un métal noble de la famille du platine, au moins un métal promoteur et un halogène, le procédé étant caractérisé en ce que la poudre d'alumine est soumise à une peptisation au moyen d'une solution aqueuse ou d'une solution aqueuse acidulée en vue de lui conférer un taux de peptisation d'au moins 10% et en ce que simultanément, au cours de la peptisation, une partie au moins de la phase active, renfermant au moins une partie du métal promoteur, est introduite dans l'alumine.

2. Procédé selon la revendication 1 dans lequel la poudre d'alumine utilisée comme support est agglomérée en présence d'une solution aqueuse ou d'une solution aqueuse acide, avec ajout simultané d'une partie au moins de la phase active, soit par extrusion soit par la méthode dite du granulateur tournant, ladite partie de phase active étant introduite sous forme soluble soit dans la solution aqueuse, soit dans la poudre, soit à la fois en partie dans la solution aqueuse et en partie dans la poudre.

3. Procédé selon la revendication 2 dans lequel la poudre d'alumine est agglomérée par extrusion, le procédé se caractérisant ainsi en ce que:

[α] pendant une durée comprise entre environ cinq minutes et cinq heures, on malaxe la poudre d'alumine en presence d'eau ou d'eau acidulée, l'eau ou l'eau acidulée renfermant ou moins en partie la phase active, la quantité d'eau ou d'eau acidulée représentant en poids 3 à 60% par rapport à la masse de la poudre,

[β] la pâte obtenue est extrudée,

[γ] les extrudés obtenus sont séchés à une température inférieure à 550°C de façon à ce que la perte au feu des solides obtenus soit de l'ordre de 2 à 40%,

[δ] les extrudés obtenus sont calcinés entre 350 et 1000°C environ.

4. Procédé selon la revendication 2 dans lequel la poudre d'alumine est agglomérée par la méthode dite du granulateur tournant, le procédé se caractérisant ainsi en ce que,

[α] d'une part on fait couler la poudre sur le granulateur et simultanément d'autre part on introduit sur le granulateur, soit en la versant soit en la vaporisant, une solution aqueuse on une solution aqueuse acidulée qui renferme au moins en partie la phase active du catalyseur,

[β] on enlève les particules qui se sont formées,

[γ] lesdites particules sont séchées à une température inférieure à 350°C de façon à ce que la perte au feu des produits obtenus soit de l'ordre de 15 à 40%.

[δ] on calcine les particules obtenues à une température comprise entre 350 et 1000°C.

5. Procédé selon l'une des revendications 2 à 4 dans lequel on utilise une alumine que l'on peptise jusqu'à un taux de peptisation compris entre 10 et 30%.

6. Procédé selon la revendication 4 dans lequel, en cours de l'étape (α), une partie au moins de la poudre, représentant 0 à 40% en poids de la totalité de la poudre utilisée, est préalablement diluée dans la solution aqueuse ou dans la solution aqueuse acidulée.

7. Procédé selon l'une des revendications 1 à 6 dans lequel on utilise un support à base d'une poudre d'alumine de haute pureté, laquelle alumine est un sous produit des réactions de synthèse de Ziegler pour la formation d'alcools supérieurs.

8. Procédé selon l'une des revendications 2 à 6 caractérisé en ce que la poudre d'alumine, avant d'être agglomérée en présence d'une solution aqueuse ou aqueuse acidulée, est d'abord divisée en deux fractions dont l'une est calcinée et mélangée avec la fraction de poudre non calcinée.

9. Procédé selon la revendication 8 dans lequel on calcine 20 à 80% en poids de la poudre d'alumine et on mélange la poudre calcinée à la poudre non calcinée de façon à obtenir, après mélange, une poudre qui se caractérise par des grains possédant un diamètre de particules de l'ordre de 1 à 50 µm.

10. Procédé selon l'une des revendications 3 et 4 dans lequel, avant de calciner les extrudés ou particules (étape γ), on procède à un traitement à l'hydrogène de ces extrudés ou particules.

11. Procédé selon l'une des revendications 1 à 10 dans lequel le catalyseur renferme en poids par rapport à la matrice ou au support d'alumine 0,02 à 1% de platine, 0,02 à 1% d'iridium, à titre de promoteur, et 0,1 à 10% d'au moins un halogène.

12. Procédé selon l'une des revendications 1 à 10 dans lequel le catalyseur renferme en poids par rapport à la matrice ou au support d'alumine (a) 0,02 à 1% d'au moins un métal précieux ou noble de la famille du platine, (b) 0,01 à 2% d'au moins un métal additionnel et (c) o,1 à 10% d'au moins un halogène.

13. Procédé selon la revendication 12 dans lequel le ou les métaux additionnels sont choisis dans le groupe constitué, par le titane, le rhénium, l'étain, le germanium, l'indium, le thallium, le manganèse, le nickel, le fer, le cobalt, le zinc, le cuivre, l'or, l'argent, le niobium, le lanthane, le cérium, le samarium, le zirconium, le thorium, l'hafnium, le plomb, le gallium, le vanadium, le téchnétium, l'uranium et le sélénium.

14. Catalyseur susceptible d'être obtenu par le procédé selon l'une des revendications 1 à 13.

15. Utilisation des catalyseurs selon la revendication 14 ou préparés selon l'une des revendications 1 à 13, dans les réactions de réformage catalytique, de production d'hydrocarbures aromatiques, d'isomérisation des hydrocarbures paraffiniques et aromatiques, d'hydrocraquage, d'hydrodéalkylation et de déalkylation à la vapeur d'eau des hydrocarbures aromatiques.

## Patentansprüche

1. Verfahren zum Herstellen eines Katalysators, der enthält: (a) eine Matrix oder einen Träger auf der Basis von Aluminiumoxidpulver und (b) eine aktive Phase, die wenigstens ein Edelmetall aus der Familie des Platins, ein Promotormetall sowie ein Halogen umfaßt, dadurch gekennzeichnet, daß das Aluminiumoxidpulver einer Peptisierung mittels einer wässrigen Lösung oder einer angesäuerten wässrigen Lösung ausgesetzt wird, um ihm einen Peptisierungsgrad von wenigstens 10 % zu verleihen und gleichzeitig während der Peptisierung wird ein Teil wenigstens der aktiven Phase, die wenigstens einen Teil des Promotormetalls umfaßt, auf das Aluminiumoxid geleitet.

2. Verfahren nach Anspruch 1, dadurch gekennzeichnet, daß das als Träger verwendete Aluminiumoxidpulver in Anwesenheit einer wässrigen Lösung oder einer sauren wässrigen Lösung unter gleichzeitigem Zusatz wenigstens eines Teils der aktiven Phase entweder durch Extrudieren oder nach dem Verfahren mit dem sog. sich drehenden Granulator agglomeriert wird, wobei dieser Teil der aktiven Phase in löslicher Form entweder in die wässrige Lösung oder in das Pulver oder gleichzeitig zum Teil in die wässrige Lösung und zum Teil in das Pulver eingeführt wird.

3. Verfahren nach Anspruch 2, wobei das Aluminiumoxidpulver durch Extrudieren agglomeriert wird, dadurch gekennzeichnet, daß

[α] über einen Zeitraum, der zwischen etwa 5 Minuten und fünf Stunden liegt, das Aluminiumoxidpulver in Anwesenheit von Wasser oder gesäuertem Wasser durch Kneten gemischt wird, wobei das Wasser oder das angesäuerte Wasser wenigstens zum Teil die aktive Phase enthält und die Menge an Wasser oder gesäuertem Wasser 3 bis 60 Gew.-% bezogen auf die Pulvermasse ausmacht,

[β] die erhaltene Paste extrudiert wird,

[γ] die erhaltenen Extrudate bei einer Temperatur unterhalb 550°C derart getrocknet werden, daß der Brennverlust der erhaltenen Feststoffe in der Größenordnung von 2 bis 40 % liegt, und

[δ] die erhaltenen Extrudate zwischen etwa 350 und 1000°C kalziniert werden.

4. Verfahren nach Anspruch 2, wobei das Aluminiumoxidpulver nach dem sog. Verfahren mit sich drehendem Granulator agglomeriert wird, dadurch gekennzeichnet, daß man

[α] einerseits das Pulver veranlaßt auf den Granulator zu fließen und gleichzeitig andererseits auf den Granulator, entweder durch Aufgießen oder durch Verdampfen eine wässrige Lösung oder eine wässrige gesäuerte Lösung gibt, die wenigstens zum Teil die aktive Phase des Katalysators enthält,

[β] die Partikel, die sich gebildet haben, entfernt,

[γ] diese Partikel bei einer Temperatur unterhalt 350°C derart trocknet, daß der Brennverlust der erhaltenen Produkte in der Größenordnung von 15 bis 40 % beträgt, und

[δ] die erhaltenen Produkte bei einer Temperatur zwischen 350 und 1000°C kalziniert.

5. Verfahren nach einem der Ansprüche 2 bis 4, dadurch gekennzeichnet, daß man ein Aluminiumoxid verwendet, das man bis zu einem Peptisierungsgrad zwischen 10 und 30 % peptisiert.

6. Verfahren nach Anspruch 4, dadurch gekennzeichnet, daß während der Stufe (α) wenigstens ein Teil des Pulvers, das 0 bis 40 Gew.-% der Gesamtheit des verwendeten Pulvers ausmacht, vorher in der wässrigen Lösung oder in der angesäuerten wässrigen Lösung verdünnt wird.

7. Verfahren nach einem der Ansprüche 1 bis 6, dadurch gekennzeichnet, daß man einen Träger auf der Basis eines Aluminiumoxidpulvers hoher Reinheit verwendet, wobei dieses Aluminiumoxid ein Nebenprodukt der Ziegler Synthesereaktionen zur Bildung höherer Alkohole ist.

8. Verfahren nach einem der Ansprüche 2 bis 4, dadurch gekennzeichnet, daß das Aluminiumoxidpulver, bevor es in Anwesenheit einer wässrigen Lösung oder einer angesäuerten wässrigen Lösung agglomeriert wird, zunächst in zwei Fraktionen geteilt wird, von denen die eine kalziniert und mit der Fraktion des nicht-kalzinierten Pulvers vermischt wird.

9. Verfahren nach Anspruch 8, wobei man 20 bis 80 Gew.-% Aluminiumoxidpulver kalziniert und man das kalzinierte Pulver mit dem nicht-kalzinierten Pulver mischt, derart, daß man nach dem Mischen ein Pulver erhält, das sich durch Körner auszeichnet, die einen Partikeldurchmesser in der Größenordnung von 1—50 µm haben.

10. Verfahren nach einem der Ansprüche 3 oder 4, dadurch gekennzeichnet, daß man vor dem Kalzinieren der Extrudate oder Partikel (Stufe γ) eine Wasserstoffbehandlung dieser Extrudate oder Partikel vornimmt.

11. Verfahren nach einem der Ansprüche 1 bis 10, dadurch gekennzeichnet, daß der Katalysator in Gew.-%, bezogen auf die Matrix oder den Träger aus Aluminiumoxid, 0,02 bis 1 % Platin, 0,02 bis 1 % Iridium als Promotor und 0,1 bis 10 Gew.-% wenigstens eines Halogens enthält.

12. Verfahren nach einem der Ansprüche 1 bis 10, dadurch gekennzeichnet, daß der Katalysator in Gew.-%, bezogen auf die Matrix oder den Träger aus Aluminiumoxid, (a) 0,02 bis 1 % wenigstens eines wertvollen oder edlen Metalls der Platinfamilie, (b) 0,01 bis 2 % wenigstens eines Zusatzmetalls und (c) 0,1 bis 10 % wenigstens eines Halogens umfaßt.

13. Verfahren nach Anspruch 12, dadurch gekennzeichnet, daß das oder die Zusatzmetalle gewählt sind aus der Gruppe, die gebildet wird durch Titan, Rhenium, Zinn, Germanium, Indium, Thallium, Mangan, Nickel, Eisen, Kobalt, Zink, Kupfer, Gold, Silber, Niob, Lanthan, Cer, Samarium, Zirkonium, Thorium, Hafnium, Blei, Gallium, Vanadium, Technezium, Uran und Selen.

14. Katalysator erhältlich nach dem Verfahren eines der Ansprüche 1 bis 13.

15. Anwendung der Katalysatoren nach Anspruch 14 oder hergestellt nach einem der Ansprüche 1 bis 13 in den Reaktionen des katalytischen Reformierens, der Herstellung aromatischer Kohlenwasserstoffe, der Isomerierung der paraffinischen und aromatischen Kohlenwasserstoffe, des Hydrokrackens, der Hydrodesalkylierung une der Desalkylierung mit Wasserdampf der aromatischen Kohlenwasserstoffe.

## Claims

1. A process for manufacturing a catalyst comprising (a) a matrix or a carrier whose base consists of alumina powder and (b) an active phase comprising at least one noble metal of the platinum family, at least one metal promoter and one halogen, the process being characterized in that the alumina powder is subjected to peptization with an aqueous solution or an acidified aqueous solution, to confer it a peptization rate of at least 10 %, and in that simultaneously, in the course of the peptization, at least a portion of the active phase comprising at least a portion of the metal promoter, is introduced into the alumina.

2. A process according to claim 1, wherein the alumina powder used as carrier is agglomerated in the presence of an aqueous solution or of an acidified aqueous solution, with simultaneous addition of at least one part of the active phase, either by extrusion or by the so-called bowl granulator method, said part of the active phase being introduced in soluble form either into the aqueous solution or into the powder, or partly into the aqueous solution and partly into the powder.

3. A process according to claim 2, wherein the alumina powder is agglomerated by extrusion, the process being characterized in that:

[α] the alumina powder is malaxed in the presence of water or acidified water for about 5 minutes to 5 hours, the water or the acidified water comprising at least a portion of the active phase, the proportion of water or acidified water amounting to 3—60 % b.w. of the powder amount,

[β] the resultant paste is extruded,

[γ] the resultant extrudates are dried at a temperature lower than 550°C, the fire loss of the resultant solids being about 2 to 40 %,

[δ] the resultant extrudates are calcined at about 350 to 1000°C.

4. A process according to claim 2, wherein the alumina powder is agglomerated by the so-called bowl granulator method, the process being thus characterized in that:

[α] simultaneously, on the one hand, the powder is introduced into the granulator and, on the other hand, an aqueous solution or an acidified aqueous solution is introduced into the granulator, either by pouring it or by vaporization, said solution containing at least a portion of the active phase of the catalyst,

[β] the formed particles are removed,

[γ] said particles are dried at a temperature lower than 350°C, the fire loss of the resultant products amounting to about 15 to 40 %,

[δ] the resultant particles are calcined at a temperature between 350 and 1000°C.

5. A process according to one of claims 2 to 4, wherein the used alumina is peptized up to a peptization capacity of 10 to 30 %.

6. A process according to claim 4, wherein, in the course of step [α], at least a portion of the powder, amounting to 0—40 % by weight of the whole powder amount to be used, is previously diluted in the aqueous solution or in the acidified aqueous solution.

7. A process according to claim 1, wherein there is used a carrier whose base is an alumina powder of high purity, said alumina being a by-product of the Ziegler synthesis reactions for manufacturing higher alcohols.

8. A process according to one of claims 2 to 6, characterized in that the alumina powder, before agglomeration in the presence of an aqueous solution or of an acidified aqueous solution is first divided in two fractions, one of them being calcined and admixed with the fraction of uncalcined powder.

9. A process according to claim 8, wherein 20 to 80 % by weight of the alumina powder is calcined and the calcined powder is admixed with the uncalcined powder so as to obtain, after admixing, a powder characterized by particles having a diameter of about 1 to 50 µm.

10. A process according to one of claims 3 or 4, wherein, before calcination of the extrudates or particles (step γ), these extrudates or particles are treated with hydrogen.

11. Process according to one of claims 1 to 10, wherein the catalyst comprises, by weight with respect to the matrix or to the alumina carrier, 0.02 to 1 % of platinum, 0.02 to 1 % of iridium, as promoter, and 0.1 to 10 % of at least one halogen.

12. Process according to one of claims 1 to 10, wherein the catalyst comprises, by weight with respect to the alumina matrix of carrier, (a) 0.02 to 1 % of at least one precious or noble metal of the platinum family, (b) 0.01 to 2 % of at least one additional metal as promoter and (c) 0.1 to 10 % of at least one halogen.

13. A process according to claim 12, wherein the additional metal(s) are selected from the group consisting of titanium, rhenium, tin, germanium, indium, thallium, manganese, nickel, iron, cobalt, zinc, copper, gold, silver, niobium, lanthanum, cerium, samarium, zirconium, thorium, hafnium, lead, gallium, vanadium, technetium, uranium and selenium.

14. Catalyst obtained by a process according to one of claims 1 to 13.

15. The use of the catalysts according to claim 14 or prepared according to one of claims 1 to 13, in the reactions of catalytic reforming, aromatic hydrocarbons production, paraffinic and aromatic hydrocarbons isomerization, hydrocracking, hydrodealkylation and steam dealkylation of aromatic hydrocarbons.